# EUROPEAN PATENT APPLICATION

(11) **EP 2 113 259 A2**
(43) Date of publication of application: **04.11.2009**
(21) Application number: 09004960.2
(22) Date of filing: 03.04.2009
(51) Int. Cl.: A61L 9/12

(54) **Fragrance dispenser**

(30) Priority: 25.04.2008 GB 0807537
(71) Applicant: Vectair Systems Limited, Kingsland Business Park Basingstoke Hampshire RG24 8LJ (GB)
(72) Inventor: Wonnacott, Paul, Esher, KT10 8LH (GB); Teeling, Matthew, 44 Richmond Road Kingston Upon Thames KT2 5EE (GB)
(74) Representative: Downing, Michael Philip

(57) **Abstract**

Existing fragrance dispensers are prone to allowing the wick to dry out too quickly. This causes the fragrance formulae to evaporate more quickly than is desired. In our arrangement we provide a fan for directing airflow past the wick, that can be driven intermittently. In a preferred arrangement, the fan has a plurality of settings offering different on/off ratios and times, and can therefore be adjusted for fragrance performance and the affects of the ambient temperatures. The fan can also have different settings offering different flow rates such as high, medium and low fan speeds. The intermittent fan option also has the advantage of considerably increasing the life of the battery from which the necessary electrical power is drawn. We have also designed an interlocking hydrophilic porous material which aids quick and easy assembly without the need for additional adhesive components. Thus, in a second aspect, the present invention provides a fragrance dispenser comprising a housing containing a removable cartridge, a motor, and a fan driven by the motor for creating an airflow past the cartridge, the cartridge containing a receptacle for fragrance and a wick comprising a sheet of porous material formed into a cylindrical shape and having interengaging formations at opposing ends of the sheet. The present invention also relates to a removable cartridge for a fragrance dispenser, as defined above.

## Description

### FIELD OF THE INVENTION

The present invention relates to a fragrance dispenser.

### BACKGROUND ART

A cartridge based fragrance reservoir dispenser. The reservoir fluid surface tension forces out weight the gravitational forces from the refill chamber. This allows the wick to reside in a constantly replenished small fragranced reservoir. Normally with these types of products there is an optional fan to assist in air flow which aid in dispersing the fragrance.

### SUMMARY OF THE INVENTION

With these types of systems there is a potential problem that the wick dries out too quickly in the airflow, or as a result of the ambient temperature being too high. This causes the fragrance formulae to evaporate more quickly than is desired. In our arrangement we provide a fan that can be driven intermittently. In a preferred arrangement, the fan has a plurality of settings offering different on/off ratios and times, and can therefore be adjusted for fragrance performance and the affects of the ambient temperatures. The fan can also have different settings offering different flow rates such as high, medium and low fan speeds.

The intermittent fan option also has the advantage of considerably increasing the life of the battery from which the necessary electrical power is drawn.

Thus, in a first aspect the present invention provides a fragrance dispenser comprising a source of fragrance in liquid form, a wick supplied by the source, a fan for directing a flow of air past the wick, a motor for the fan, and a control means for controlling the motor, wherein the control means is adapted to drive the motor intermittently.

The control means can be adapted to drive the motor for a first preset period and rest the motor for a second preset period. The first preset period is preferably less than the second preset period. This can then be repeated indefinitely. It can stop when an operator intervenes or when some other event causes it to stop, such as exhaustion of a battery supplying power to the motor and/or the control unit or a contrary instruction in the programming of the control unit.

We have also designed an interlocking Hydrophilic porous material which aids quick and easy assembly without the need for additional adhesive components.

Thus, in a second aspect, the present invention provides a fragrance dispenser comprising a housing containing a removable cartridge, a motor, and a fan driven by the motor for creating an airflow past the cartridge, the cartridge containing a receptacle for fragrance and a wick comprising a sheet of porous material formed into a cylindrical shape and having interengaging formations at opposing ends of the sheet.

The present invention also relates to a removable cartridge for a fragrance dispenser, as defined above.

### BRIEF DESCRIPTION OF THE DRAWINGS

An embodiment of the present invention will now be described by way of example, with reference to the accompanying figures in which;
Figure 1 shows the fragrance dispenser of the present invention, closed;
Figure 2 shows the fragrance dispenser of the present invention, open and partially assembled;
Figure 3 shows the fan assembly for fitting to the dispenser of figure 2;
Figure 4 shows the settings of the fan assembly;
Figure 5 shows an instruction label for the fan settings;
Figure 6 shows the wick in flat form prior to assembly;
Figure 7 shows a vertical section through the cartridge; and
Figure 8 shows a vertical section through the complete dispenser.

### DETAILED DESCRIPTION OF THE EMBODIMENTS

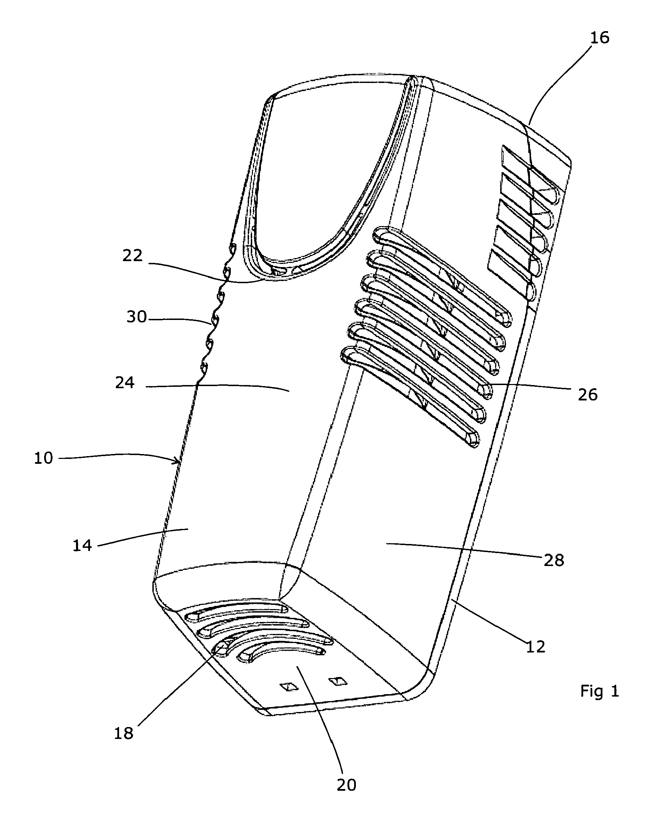

Referring to figure 1, a fragrance dispenser 10 according to the present invention comprises a rear panel 12 which will usually be attached to a wall at a suitable elevated position, and a front cover 14 which is hinged to the rear panel 12 at a top edge 16. The front cover 14 defines a box section which extends forward from the rear panel 12, to define an interior space in which the working elements of the fragrance dispenser can be placed. Air vents are distributed over the surface of the front cover 14 and are positioned so as to allow the desired air flow into and out of the dispenser. Thus, a series of slits 18 are located on the lower face 20 of the front cover 14 to allow ingress of air. In addition, a large decoratively-shaped slit 22 is placed on the front face 24 of the front cover 14, and a series of slits 26 are placed on a side face 28 of the front cover (duplicated 30 on the other opposing side face that is not visible in figure 1).

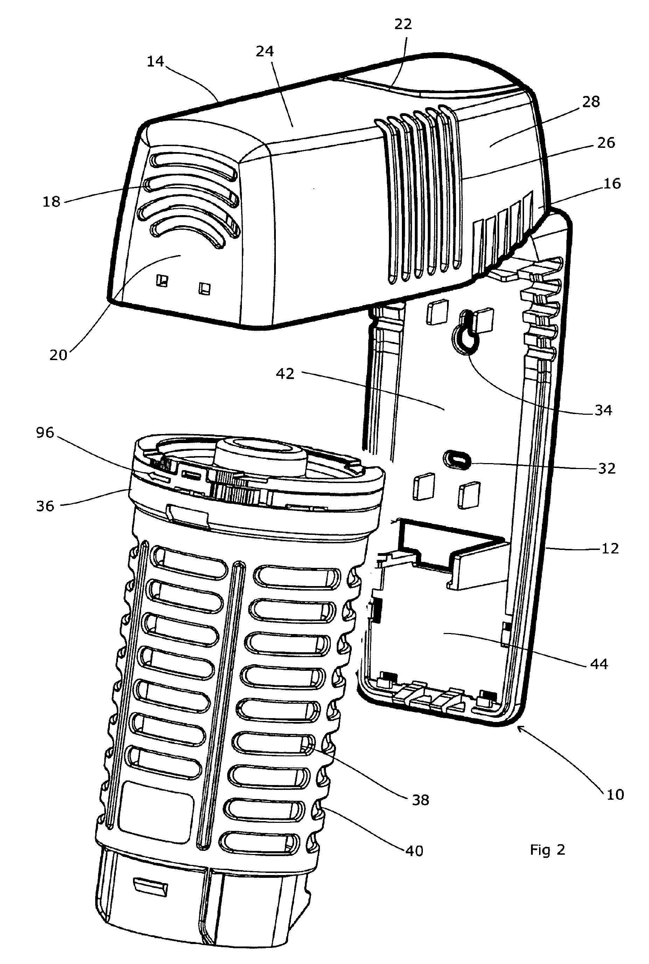

Figure 2 shows the fragrance dispenser 10 open, with the front cover 14 hinged away from the rear panel 12 via the hinge at the top edge 16. The hinge is placed at the top edge of the dispenser 10 so that an operator can easily access the interior of the dispenser 10 despite the fact that it will usually be mounted at an elevated position. Thus, the operator will usually approach the dispenser from below. Figure 2 shows attachment holes 32, 34 on the rear panel to allow it to be affixed to a wall, together with various lugs and guides to allow the internal components to be fitted in place.

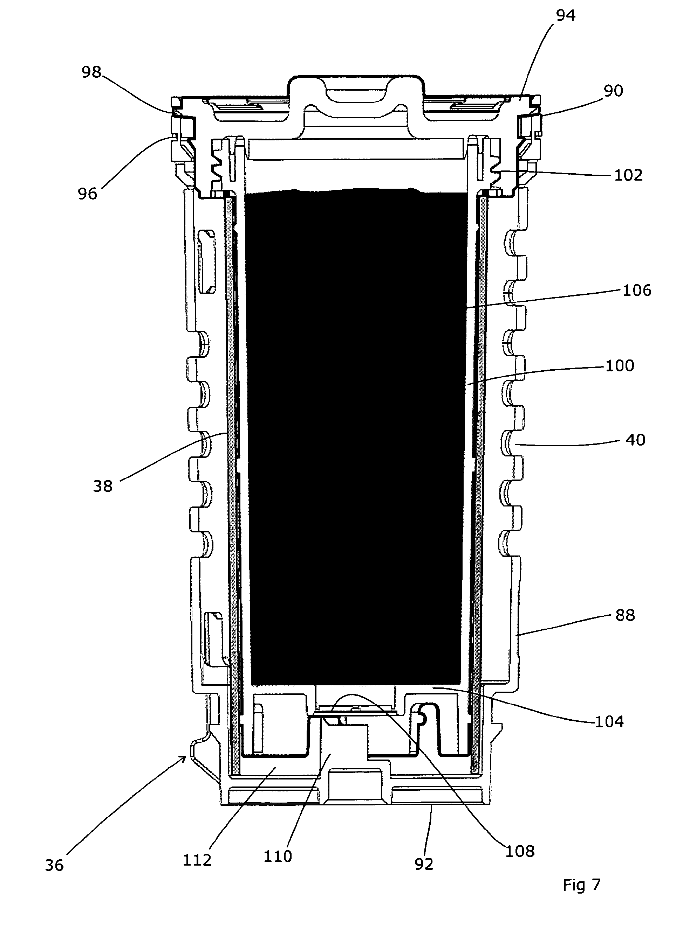

A first such internal component is a cartridge 36. This is a generally cylindrical container in which is a porous cylindrical wick 38, visible through a pattern of apertures 40 covering most of the surface of the cartridge 36. A source of fragrance is located within the cylindrical wick 38 to keep the wick 38 supplied as shown in figure 7. Thus, air flowing past the cartridge 36 can enter and leave via the pattern of apertures 40 and thus pick up fragrance from the wick 38. The cartridge can be clipped to the rear face 12 of the dispenser 10 in an upper internal region 42 thereof prior to closing the front cover 14.

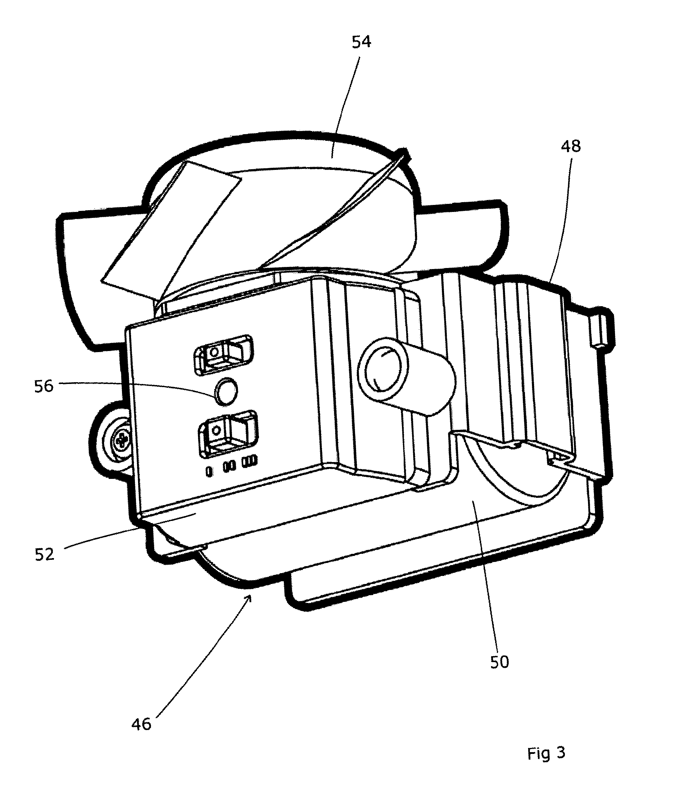

The lower internal region 44 accepts a fan unit 46 shown in figure 3. This unit 46 comprises a housing 48 that accepts a dry cell battery 50 and contains a programmed control unit and motor 52. The motor output shaft drives a fan 54 located on an upper surface of the control unit 52, positioned so that when the fan unit 46 is fitted in the dispenser on the internal side of the rear face 12, it will be directly below the cartridge 36. A switch panel 56 is provided on the front of the control unit 52 to allow an operator to place the control unit 52 in a desired mode.

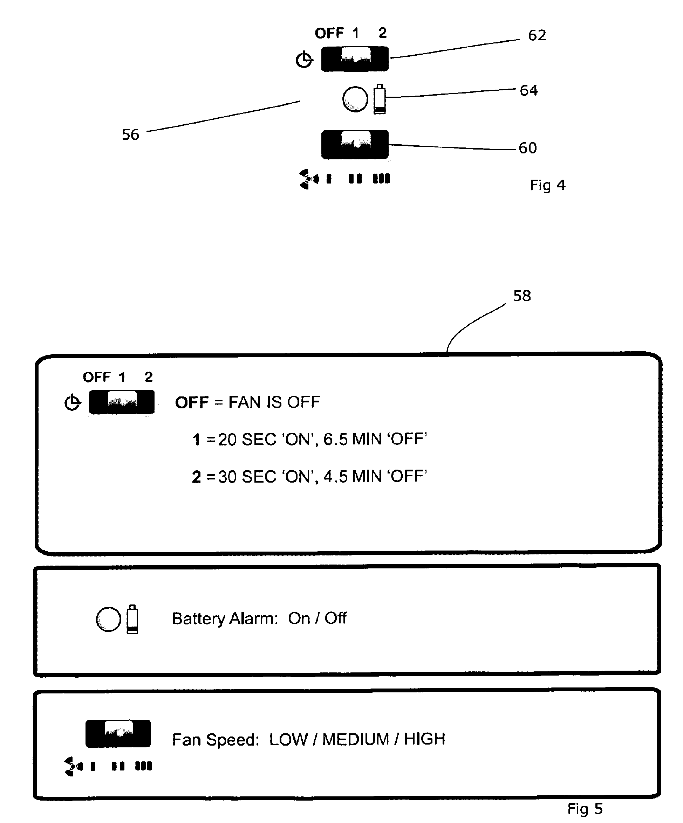

Figure 4 shows the switch panel 56 and figure 5 shows the associated label or instruction note 58 giving details of the manner of operation. As set out in the label 58, a lower slide switch 60 allows a fan speed to be set, from a choice of low, medium or high. An upper slide switch 62 places the control unit 52 in one of three modes;
- a first mode labelled as "OFF" in which the fan does not operate at all
- a second mode labelled as "1" in which the fan will operate intermittently, on for 20 seconds and then off for 6½ minutes before repeating
- a third mode labelled as "2" in which the fan will operate intermittently, on for 30 seconds and then off for 4½ minutes before repeating

Other timing patterns or combinations of times could of course be chosen as required. Control of the ratio of the on/off times allows for control of the dispensing rate, while control of the actual times allows for control of the smoothness of dispensing. If the individual times are set at too long a period, users may notice a variation in odour. However, given that electric motors require a surge of power when they start to rotate, selection of times that are too short will increase power consumption and reduce battery life. In our opinion, these times are a good compromise but others may wish to adopt a different compromise while still benefiting from the invention.

A light 64 is also provided between the two switches 60, 62, to indicate a low battery condition. It may be preferable to program the control unit 52 to default to the "OFF" mode regardless of the position of the upper slide switch 62 when a low battery condition is noted. In this way, battery power can be reserved for the light so that an operator can be alerted to the condition immediately, rather than have to (potentially) wait for 6 minutes to confirm that the motor is not running.

Thus, when operating, the fan 54 can draw air in through the slits 18 on the lower face 20 of the dispenser 10 and push it over the cartridge 36 and hence the wick 38 therewithin. The air will continue to flow up and out of the slits 22, 26, 30 on the front 24 and sides 28 of the dispenser 10, into the room around the dispenser.

In time, the fragrance within the cartridge 36 will become exhausted. At this point, the dispenser can be opened to remove the cartridge 36 and replace it with a fresh one. The old cartridge can be refilled, recycled, or disposed of. Additional cartridges can be supplied as a separate item.

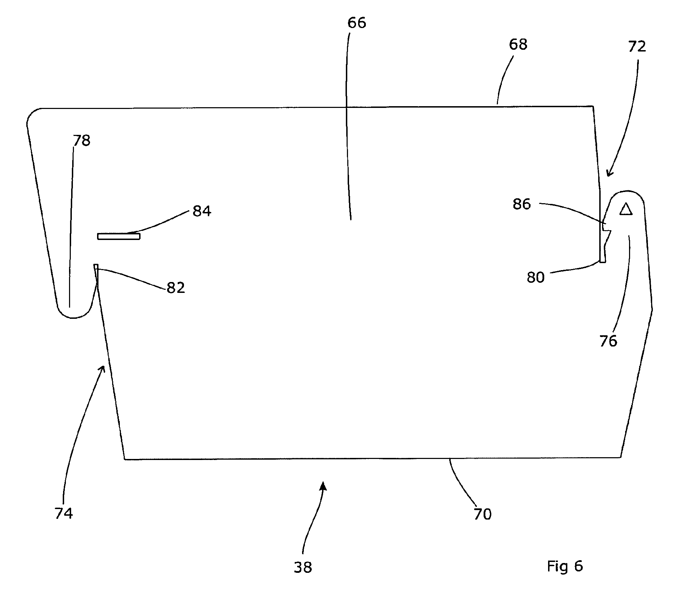

Figure 6 shows the wick 38, folded flat. It can be seen that this comprises a flat sheet 66 of a suitable absorbent paper with straight parallel upper and lower edges 68, 70. It can thus be folded around a vertical axis to form a cylindrical shape as visible in figure 2. To keep the sheet in this configuration, instead of glue or other forms of fixing, we have shaped the lateral edges 72, 74 of the sheet 66 so that each includes a tab 76, 78 that projects substantially parallel to the respective edge 72, 74 to define slit 80, 82 between the tab 76, 78 and the remainder of the edge 72, 74. Thus, after folding the sheet 66, the two slits can then be engaged to hold the sheet 66 in the folded state.

The result of this is that one tab 78 will lie inside the cylinder thus formed and one tab 76 will lie outside. To prevent this outer tab from touching the cartridge 36, and to retain the engagement of the slits 80, 82, a slot 84 is provided into which the outer tab 76 can be fitted. The tab 76 is provided with a sawtooth formation 86 along one edge in order to hinder its removal from the slot 84 and assist in ensuring that the cylinder remains the correct shape.

By forming the wick in this way, the expense and time required in assembling a wick using glue or other fixings is avoided, and the production costs can be minimised.

Figure 7 shows a section through the cartridge 36 incorporating this wick. The external shell 88 of the cartridge 36 defines a generally cylindrical shape with an open circular top 90 and a closed circular base 92. A lid 94 fits snugly into the open top 90 of the shell 88, and is shown almost "home" in figure 7. Initially (as shown), the lid 94 is prevented from being pushed fully home by a lock strip 96; this sits between a rim 98 of the lid 94 and the open top 90 and acts as a spacer. Immediately prior to use, the lock strip 96 is removed by an operator and the lid 94 can then be pressed fully home.

A fragrance container 100 is screwed to the underside of the lid 94 via cooperating screw threads 102. The container 100 is generally cylindrical, albeit smaller than the shell 88 and hence able to fit inside. It has a closed base 104 and an open top that is sealed by the lid 94. The interior of the container 100 is filled with a blended fragrance fluid 106.

A fragile rupture panel 108 is provided in the closed base 104 of the container 100. Immediately below this, there is a puncturing member 110 which extends upwardly from the closed circular base 92 of the shell 88. As shown in figure 7, with the lid 94 and container 100 being prevented from being pushed fully home by the lock strip 96, the puncturing member 110 ends just short of the rupture panel 108. When the lock strip 96 is removed and the lid 94 and container 100 are pushed fully home, the puncturing member 110 will pierce the rupture panel 108 and allow the fragrance fluid 106 to flow into a reservoir 112 around the puncturing member 110.

This will permit the fragrance fluid 106 to contact the cylindrical wick 38 that is located within the shell 88, around the container 100. Fragrance fluid 106 will then be taken up by the porous material of the wick 38 and can be imparted to air flowing past the cartridge 36 via the pattern of apertures 40.

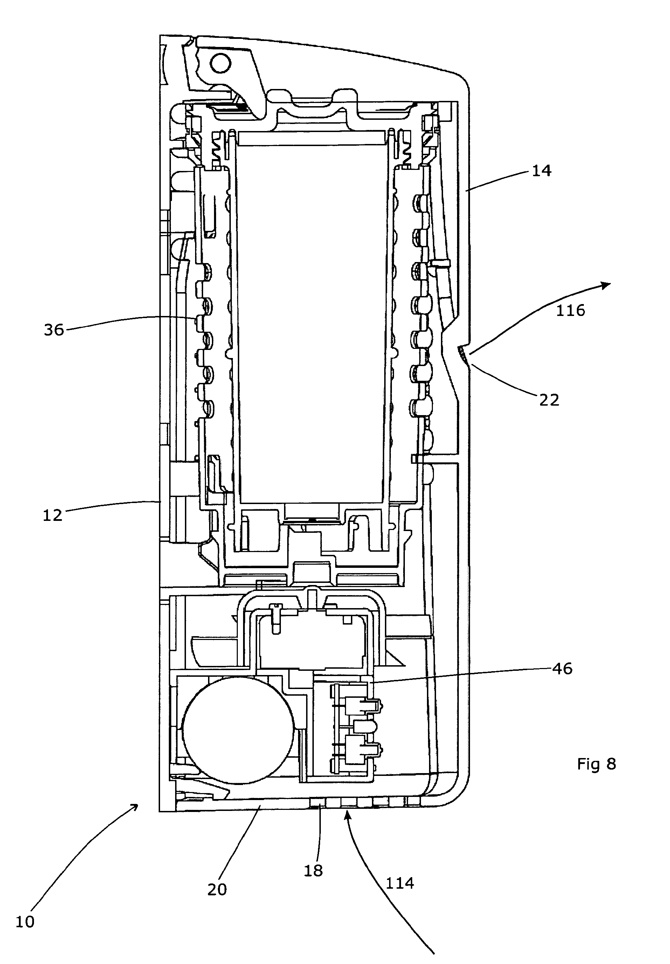

Figure 8 shows the complete dispenser 10 with the front cover 14 closed and the rear panel 12 vertical as if fixed to a wall. The cartridge 36 is fitted within the dispenser 10, immediately above the fan unit 46. Air will be drawn in through the slits 18 on the lower face 20 of the front cover 14 as shown by arrow 114 and directed past the cartridge 36 before exiting via the slits 22, 26, 30 on the front 24 and sides 28 of the front cover 14 as shown by arrow 116.

It will of course be understood that many variations may be made to the above-described embodiment without departing from the scope of the present invention.

## Claims

1. A fragrance dispenser comprising a source of fragrance in liquid form, a wick supplied by the source, a fan for directing a flow of air past the wick, a motor for the fan, and a control means for controlling the motor, wherein the control means is adapted to drive the motor intermittently.

2. A fragrance dispenser according to claim 1 in which the control means is adapted to drive the motor for a first preset period and rest the motor for a second preset period.

3. A fragrance dispenser according to claim 2 in which the first preset period is less than the second preset period.

4. A fragrance dispenser according to claim 2 or claim 3 in which the control means is adapted to repeat the cycle indefinitely.

5. A fragrance dispenser comprising a housing containing a removable cartridge, a motor, and a fan driven by the motor for creating an airflow past the cartridge, the cartridge containing a receptacle for fragrance and a wick comprising a sheet of porous material formed into a cylindrical shape and having interengaging formations at opposing ends of the sheet.

6. A removable cartridge for a fragrance dispenser, comprising a receptacle for fragrance and a wick comprising a sheet of porous material formed into a cylindrical shape and having interengaging formations at opposing ends of the sheet.
